(19) Europäisches Patentamt
European Patent Office
Office européen des brevets

(11) Veröffentlichungsnummer : **0 246 189 B1**

(12) **EUROPÄISCHE PATENTSCHRIFT**

(45) Veröffentlichungstag der Patentschrift : **11.03.92 Patentblatt 92/11**

(51) Int. Cl.$^5$ : **C08K 5/32**, C08K 5/34, C07C 239/00, C07D 213/04, C07D 295/12

(21) Anmeldenummer : **87810285.4**

(22) Anmeldetag : **06.05.87**

(54) **Mit Aminoxyalkylaminen stabilisierte Zusammensetzungen.**

(30) Priorität : **12.05.86 US 861960**

(43) Veröffentlichungstag der Anmeldung : **19.11.87 Patentblatt 87/47**

(45) Bekanntmachung des Hinweises auf die Patenterteilung : **11.03.92 Patentblatt 92/11**

(84) Benannte Vertragsstaaten : **BE DE FR GB IT NL**

(56) Entgegenhaltungen :
**TETRAHEDRON LETTERS, Nr. 29, 1974, Seiten 2493-2496, Pergamon Press, GB; C. BERNHART et al.: "Reduction D'O-alkyl benzaldoximes en O-alkyl hydroxylamines"**
**CHEMICHE BERICHTE; Teil 99, 1966, Seiten 895-902; G. ZIMMER et al.: "Über Acetale mit N-Hydroxy-dialkylaminen"**

(73) Patentinhaber : **CIBA-GEIGY AG**
**Klybeckstrasse 141**
**CH-4002 Basel (CH)**

(72) Erfinder : **Ravichandran, Ramanathan**
**111 De Haven Drive, Apt. 125**
**Yonkers New York 10703 (US)**
Erfinder : **Pastor, Stephen D.**
**1080 Warburton Avenue. Apt. 1C**
**Yonkers New York 10701 (US)**
Erfinder : **Snead, Thomas E.**
**39 King Street**
**Dobbs Ferry New York 10522 (US)**

**EP 0 246 189 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft mit Aminoxyalkylaminen gegen thermischen, oxidativen und/oder aktinischen Abbau stabilisiertes organisches Material, die Verwendung von diesen Aminoxyalkylaminen sowie neue Aminoxyalkylaminderivate.

Organische Materialien, wie z.B. Kunststoffe und Harze, unterliegen thermischem, oxidativem und lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise die Wirksamkeit eines Stabilisators, der die Flüchtigkeit einer Verbindung reduziert, darauf zurückgeführt werden, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenabbruch verhindert. Um die Vergilbung zu unterbinden, müssen Reaktionen, die zu neuen Chromophoren führen, verhindert werden. Weiterhin müssen Probleme der Verarbeitungsstabilität und der Substratverträglichkeit beachtet werden.

Verschiedene organische Hydroxylamine sind bekannt und einige sind im Handel erhältlich. Eine Anzahl von Patenten offenbaren substituierte Hydroxylamine als Antioxidantien für verschiedene Substrate, einschliesslich Polyolefine, Polyester und Polyurethane. Als repräsentative Patente seien die US-Patente 3 432 578, 3 644 278, 3 778 464, 3 408 422, 3 926 909, 4 316 996 und 4 386 224 genannt, welche im wesentlichen N,N-Dialkyl-, N,N-Diaryl- und N,N-Diaralkylhydroxylamine und deren Wirksamkeit als Farbverbesserer beschreiben.

Ferner beschreiben Zinner et al in "Chem. Berichte 99, 895 (1966)" eine Mannich-Kondensation von bestimmten N-Hydroxy-dialkylaminen, Formaldehyd und sekundären Aminen. Viele der erhaltenen Verbindungen besitzen ein niedriges Molekulargewicht. Typische Formeln schliessen

$$R_2NOCH(R)NR'_2$$

und $(R_2NOCH_2)_2N-CH_3$ ein, wobei die verschiedenen R's Niederalkyl, Benzyl, Gyclohexyl und einen heterocyclischen Rest bedeuten. Es wird keine praktische Verwendung für diese Verbindungen angegeben. Bernhart et al offenbaren in "Tetrahedron Letters 29, 2493-2496 (1974)" die Verbindung $C_6H_5CH_2-NH-OCH_2CH_2N(C_2H_5)_2$ und deren pharmakologische Verwendung.

Die vorliegende Erfindung betrifft Zusammensetzungen enthaltend ein gegen oxidativen, thermischen und/oder aktinischen Abbau empfindliches organisches Material und mindestens eine Verbindung der Formel I,

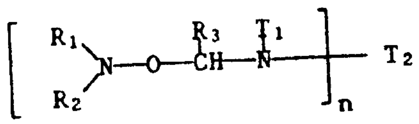

(I)

worin $R_1$ und $R_2$ unabhängig voneinander $C_1$-$C_{36}$-Alkyl, $C_5$-$C_{12}$ -Cycloalkyl, $C_7$-$C_9$-Aralkyl oder durch $C_1$-$C_{36}$-Alkyl substituiertes $C_7$-$C_9$-Aralkyl bedeuten oder $R_1$ und $R_2$ zusammen mit dem Stickstoffatom, an das sie gebunden sind, einen 5- bis 7-gliedrigen heterocyclischen Ring bilden oder $R_1$ und $R_2$ unabhängig voneinander eine Gruppe der Formel (II)

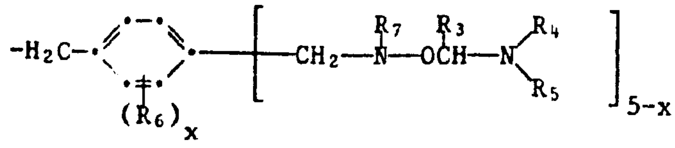

(II)

sind, $R_3$, $R_4$, $R_5$, $R_6$ und $R_7$ unabhängig voneinander Wasserstoff, $C_1$-$C_{36}$-Alkyl, $C_5$-$C_{12}$-Cycloalkyl, Allyl, Aryl, $C_7$-$C_9$-Aralkyl, durch $C_1$-$C_{36}$-Alkyl substituiertes $C_7$-$C_9$-Aralkyl, Bornyl oder Norbornyl darstellen oder $R_4$ und $R_5$ zusammen mit dem Stickstoffatom, an das sie gebunden sind, einen 5- bis 7-gliedrigen heterocyclischen Ring bilden, x eine ganze Zahl von 0 bis 5 und n von 1 bis 4 bedeuten, $T_1$ eine der für $R_3$ angegebenen Bedeutungen besitzt oder

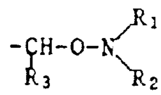

ist,

wenn n 1 ist, $T_2$ eine der für $R_3$ angegebenen Bedeutungen hat oder $C_1$-$C_4$-Alkyl, welches durch einen 5- bis 7-gliedrigen, ungesättigten heterocyclischen Ring substituiert ist, bedeutet oder die Reste $T_1$ und $T_2$ zusammen mit dem Stickstoffatom, an das sie gebunden sind, einen 5- bis 7-gliedrigen, gesättigten heterocyclischen Ring bilden,

wenn n 2 bedeutet, $T_2$ $C_2$-$C_{12}$-Alkylen, $C_6$-$C_{10}$-Cycloalkylen, $C_6$-$C_{10}$-Arylen oder $C_8$-$C_{10}$-Alkylenarylenalkylen ist oder $T_1$ und $T_2$ zusammen mit den beiden Stickstoffatomen, an die sie gebunden sind, einen 5- bis 7-gliedrigen, gesättigten heterocyclischen Ring bilden,

wenn n 3 bedeutet, $T_2$ $C_3$-$C_6$-Alkantriyl ist oder $T_1$ und $T_2$ zusammen mit den drei Stickstoffatomen, an die sie gebunden sind, einen 5- bis 6-gliedrigen, gesättigten heterocyclischen Ring bilden und

wenn n 4 bedeutet, $T_2$ $C_4$-$C_6$-Alkantetrayl darstellt.

Die Zusammensetzungen dieser Erfindung weisen verschiedene wertvolle Eigenschaften auf, die auf die Anwesenheit von den oben angegebenen Aminoxyalkylaminderivaten zurückzuführen sind. So schützen diese Verbindungen eine Reihe von Substraten, wie z.B. Polyolefine, Elastomere und Schmiermittel gegen oxidativen und thermischen Abbau. Ferner sind sie sehr wirkungsvolle Farbverbesserer und Verarbeitungsstabilisatoren für Polyolefinzusammensetzungen, die Metallsalze von Fettsäuren und/oder phenolische Antioxidantien enthalten. Hauptsächlich können die Aminoxyalkylaminderivate dazu dienen, Verfärbungen zu vermindern, die auf die Gegenwart von phenolischen Antioxidantien und/oder auf die Verarbeitungsbedingungen zurückzuführen sind. Ferner schützen sie das organische Polymer direkt vor den Auswirkungen der Verarbeitungsbedingungen. Sie schützen auch solche Polyolefinzusammensetzungen, die gehinderte Amin-Licht-stabilisatoren oder Kombinationen von phenolischen Antioxidantien und Phosphiten enthalten, vor der Verfärbung.

Die Reste $R_1$ bis $R_7$ bedeuten als $C_1$-$C_{36}$-Alkyl zum Beispiel Methyl, Ethyl, n-Propyl, n-Butyl, tert-Butyl, n-Pentyl, n-Octyl, 2-Ethylhexyl, Decyl, Dodecyl oder Octadecyl. $C_1$-$C_{18}$-Alkyl, welches geradkettig oder verzweig sein kann, ist bevorzugt. Besonders bevorzugt ist $C_1$-$C_6$-Alkyl.

Die Reste $R_1$ bis $R_7$ sind als $C_5$-$C_{12}$-Cycloalkyl beispielsweise Cyclopentyl, Cyclohexyl oder Cyclooctyl. Bevorzugt ist Cycloalkyl mit 5 bis 7 Kohlenstoffatomen, insbesondere Cyclopentyl und Cyclohexyl.

Die Reste $R_1$ bis $R_7$ können als unsubstituiertes oder durch $C_1$-$C_{36}$-Alkyl substituiertes $C_7$-$C_9$-Aralkyl insbesondere Phenylalkyl mit 7 bis 9 Kohlenstoffatomen bedeuten, wobei der Phenylring gegebenenfalls durch $C_1$-$C_{36}$-Alkyl, bevorzugt Methyl, substituiert sein kann. Beispiele sind Benzyl, $\alpha$-Methylbenzyl und $\alpha,\alpha$-Dimethylbenzyl. $R_1$ und $R_2$ sind bevorzugt unsubstituiertes oder substituiertes Benzyl.

Die Reste $R_3$ bis $R_7$ bedeuten als Aryl zum Beispiel Phenyl.

Bilden die Reste $R_1$ und $R_2$ bzw. die Reste $R_4$ und $R_5$ zusammen mit dem Stickstoffatom, an das sie gebunden sind, einen 5- bis 7-gliedrigen heterocyclischen Ring, so handelt es sich beispielsweise um Pyrryl, Piperidino, Morpholino oder Pyrrolidino.

n bedeutet 1, 2, 3 oder 4, bevorzugt 1, 2 oder 3.

x bedeutet 0, 1, 2, 3, 4 oder 5, insbesondere 1 oder 2.

Beispiele für $T_1$ sind die oben für $R_3$ angegebenen Bedeutungen.

Wenn n 1 ist, bedeutet $T_2$ als $C_1$-$C_4$-Alkyl, welches durch eine ungesättigte, 5- bis 7-gliedrige heterocyclische Gruppe, bevorzugt Pyridinyl, substituiert ist, zum Beispiel 2-(Pyridin-2-yl)ethyl. Weitere Beispiele für $T_2$ sind die oben für $R_3$ angegebenen Bedeutungen.

Wenn n 1 bedeutet und die Reste $T_1$ und $T_2$ zusammen mit dem Stickstoffatom, an das sie gebunden sind, einen gesättigten, 5- bis 7-gliedrigen heterocyclischen Rest bilden, so handelt es sich beispielsweise um Pyrrolidino, Piperidino oder Morpholino.

$T_2$ bedeutet als $C_2$-$C_{12}$-Alkylen zum Beispiel Ethylen, Trimethylen, Tetramethylen, Pentamethylen, Hexamethylen oder Decamethylen. $C_2$-$C_6$-Alkylen, insbesondere geradkettiges, ist bevorzugt.

$T_2$ bedeutet als Cycloalkylen mit 6 bis 10 Kohlenstoffatomen bevorzugt Cyclohexylen und als $C_6$-$C_{10}$-Arylen zum Beispiel Phenylen.

$T_2$ bedeutet als $C_8$-$C_{10}$-Alkylenarylenalkylen bevorzugt Alkylenphenylenalkylen, insbesondere Xylylen.

Wenn n 2 ist und die Reste $T_1$ und $T_2$ zusammen mit den beiden Stickstoffatomen, an die sie gebunden sind, einen gesättigten, 5- bis 7-gliedrigen heterocyclischen Ring bilden, so handelt es sich beispielsweise um Piperazin oder Pyrazolidin.

$T_2$ bedeutet als $C_3$-$C_6$-Alkantriyl z.B. Glyceryl oder Trimethylylpropan.

Wenn n 3 ist und die Reste $T_1$ und $T_2$ zusammen mit den drei Stickstoffatomen, an die sie gebunden sind,

einen gesättigten, 5- bis 6-gliedrigen heterocyclischen Ring bilden, so handelt es sich zum Beispiel um abgesättigtes Triazin oder Triazol, insbesondere Hexahydro-1,3,5-triazin.

$T_2$ bedeutet als $C_4$-$C_6$-Alkantetrayl bevorzugt Pentaerythrityl.

$R_1$ und $R_2$ sind bevorzugt Benzyl oder Ethyl.

Wenn n 2 oder 3 bedeutet, sind typische Beispiele für heterocyclische Verbindungen der Formel I

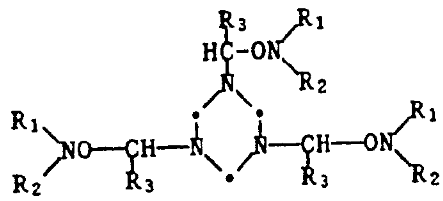

und

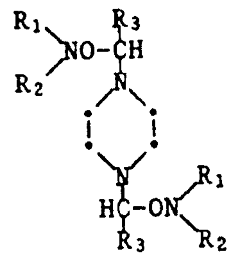

worin $R_1$, $R_2$ und $R_3$ die oben angegebenen Bedeutungen besitzen.

Die erfindungsgemässen Zusammensetzungen enthalten bevorzugt solche Verbindungen der Formel 1, worin die Reste $R_1$ bis $R_7$ unabhängig voneinander $C_1$-$C_{18}$-Alkyl, Cyclopentyl, Cyclohexyl, Benzyl, α-Methylbenzyl oder α,α-Dimethylbenzyl sind und $R_3$ zusätzlich Wasserstoff bedeutet.

Besonders bevorzugt sind Verbindungen der Formel I, worin $R_1$ und $R_2$ Benzyl sind und $R_3$ Wasserstoff bedeutet.

Ebenfalls bevorzugt sind Verbindungen der Formel I, worin n 1 ist, $T_1$ und $T_2$ unabhängig voneinander $C_1$-$C_{18}$-Alkyl, Cyclopentyl, Cyclohexyl, Allyl, Phenyl, Benzyl, α-Methylbenzyl oder α,α-Dimethylbenzyl bedeuten oder die Reste $T_1$ und $T_2$ zusammen mit dem Stickstoffatom, an das die gebunden sind, eine Morpholino-, Pyrrolidino- oder Piperidinogruppe bilden.

Wenn n 2 ist, bedeutet $T_2$ bevorzugt $C_2$-$C_6$-Alkylen, Phenylen oder Xylylen oder $T_1$ und $T_2$ bilden zusammen mit den beiden Stickstoffatomen, an die sie gebunden sind, einen Piperazinrest.

Wenn n 3 ist, bedeutet $T_2$ bevorzugt Glyceryl oder Trimethylylpropan oder $T_1$ und $T_2$ bilden zusammen mit den drei Stickstoffatomen, an die sie gebunden sind, einen Hexahydro-1,3,5-triazinring.

Wenn n 4 ist, bedeutet $T_2$ bevorzugt Pentaerythrityl.

Besonders bevorzugt sind Verbindungen der Formel I, worin $R_1$ und $R_2$ unabhängig voneinanaer $C_1$-$C_6$-Alkyl oder Benzyl sind, $R_3$ Wasserstoff ist, n 1, 2 oder 3 bedeutet und wenn n 1 ist, $T_1$ und $T_2$ unabhängig voneinander $C_1$-$C_8$-Alkyl, Allyl, Phenyl, Benzyl oder Norbornyl sind und $T_2$ zusätzlich $C_1$-$C_4$-Alkyl, welches durch eine Pyridinylgruppe substituiert ist, bedeutet oder $T_1$ und $T_2$ zusammen mit dem Stickstoffatom, an das sie gebunden sind, eine Piperidinogruppe bilden, wenn n 2 ist, $T_1$ $C_1$-$C_6$-Alkyl und $T_2$ $C_2$-$C_6$-Alkylen sind oder $T_1$ und $T_2$ zusammen mit den beiden Stickstoffatomen, an die sie gebunden sind, einen Piperazinring bilden und wenn n 3 bedeutet, $T_1$ und $T_2$ zusammen mit den drei Stickstoffatomen, an die sie gebunden sind, eine Hexahydro-1,3,5-triazingruppe bilden.

Von Interesse sind die erfindungsgemässen Zusammensetzungen, worin die Verbindung der Formel I
1,4-Bis[N,N-dibenzylaminoxymethyl]piperazin,
N′,N″-Dimethyl-N′,N″-bis[N,N-dibenzylaminoxymethyl]hexamethylendiamin,
[N,N-Dibenzylaminoxymethyl)di-n-butylamin,
[N,N-Dibenzylaminoxymethyl)di-n-hexylamin,
[N,N-Dibenzylaminoxymethyl)diisopropylamin,
[N,N-Diethylaminoxymethyl)di-n-butylamin,
[N,N-Dibenzylaminoxymethyl)-N′-methyl-N′-2-(pyridin-2-yl)ethylamin,
[N,N-Dibenzylaminoxymethyl)-N′-benzyl-N′-exo-norbornylamin oder
N′,N″,N‴-Tris[N,N-dibenzylaminoxymethyl]hexahydro-1,3,5-triazin ist.

Ein weiterer Gegenstand der Erfindung sind die neuen Verbindungen der Formel IA,

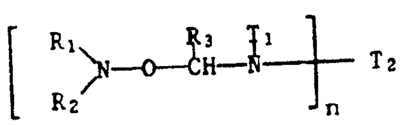

(IA)

worin n eine ganze Zahl von 1 bis 4 bedeutet, wenn n 1 ist, $R_1$ und $R_2$ unabhängig voneinander eine Gruppe

der Formel (IIA)

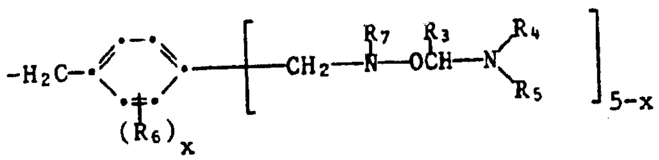

(IIA)

sind, $R_3$, $R_4$, $R_5$, $R_6$ und $R_7$ unabhängig voneinander Wasserstoff, $C_1$-$C_{36}$-Alkyl, $C_5$-$C_{12}$-Cycloalkyl, Allyl, Aryl, $C_7$-$C_9$-Aralkyl, durch $C_1$-$C_{36}$-Alkyl substituiertes $C_7$-$C_9$-Aralkyl, Bornyl oder Norbornyl darstellen oder $R_4$ und $R_5$ zusammen mit dem Stickstoffatom, an das sie gebunden sind, einen 5- bis 7-gliedrigen heterocyclischen Ring bilden, x eine ganze Zahl von 0 bis 4 bedeutet, $T_1$ eine der für $R_3$ angegebenen Bedeutungen besitzt oder

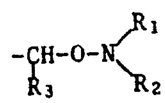

ist,

$T_2$ eine der für $R_3$ angegebenen Bedeutungen hat oder $C_1$-$C_4$-Alkyl, welches durch einen 5- bis 7-gliedrigen, ungesättigten heterocyclischen Ring substituiert ist, bedeutet oder die Reste $T_1$ und $T_2$ zusammen mit dem Stickstoffatom, an das sie gebunden sind, einen 5- bis 7-gliedrigen, gesättigten heterocyclischen Ring bilden, wenn n 2, 3 oder 4 bedeutet, $R_1$ und $R_2$ unabhängig voneinander $C_1$-$C_{36}$-Alkyl, $C_5$-$C_{12}$-Cycloalkyl, $C_7$-$C_9$-Aralkyl oder durch $C_1$-$C_{36}$-Alkyl substituiertes $C_7$-$C_9$-Aralkyl bedeuten oder $R_1$ und $R_2$ zusammen mit dem Stickstoffatom, an das sie gebunden sind, einen 5- bis 7-gliedrigen heterocyclischen Ring bilden oder $R_1$ und $R_2$ unabhängig voneinander eine Gruppe der Formel (II)

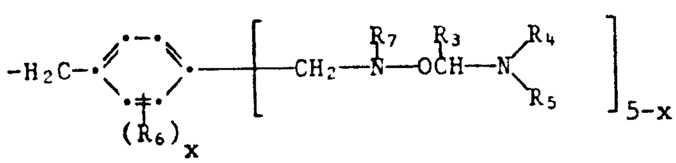

(II)

sind, $R_3$, $R_4$, $R_5$, $R_6$ und $R_7$ unabhängig voneinander Wasserstoff, $C_1$-$C_{36}$-Alkyl, $C_5$-$C_{12}$-Cycloalkyl, Allyl, Aryl, $C_7$-$C_9$-Aralkyl, durch $C_1$-$C_{36}$-Alkyl substituiertes $C_7$-$C_9$-Aralkyl, Bornyl oder Norbornyl darstellen oder $R_4$ und $R_5$ zusammen mit dem Stickstoffatom, an das sie gebunden sind, einen 5- bis 7-gliedrigen heterocyclischen Ring bilden, x eine ganze Zahl von 0 bis 5 bedeutet, $T_1$ eine der für $R_3$ angegebenen Bedeutungen besitzt oder

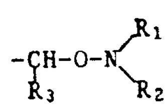

ist,

wenn n 2 bedeutet, $T_2$ $C_2$-$C_{12}$-Alkylen, $C_6$-$C_{10}$-Cycloalkylen, $C_6$-$C_{10}$-Arylen oder $C_8$-$C_{10}$-Alkylenarylenalkylen ist oder $T_1$ und $T_2$ zusammen mit den beiden Stickstoffatomen, an die sie gebunden sind, einen 5- bis 7-gliedrigen, gesättigten heterocyclischen Ring bilden,

wenn n 3 bedeutet, $T_2$ $C_3$-$C_6$-Alkantriyl ist oder $T_3$ und $T_2$ zusammen mit den drei Stickstoffatomen, an die sie gebunden sind, einen 5- bis 6-gliedrigen, gesättigten heterocyclischen Ring bilden und

wenn n 4 bedeutet, $T_2$ $C_4$-$C_6$-Alkantetrayl darstellt.

Bezüglich der Variablen $R_1$ bis $R_7$, $T_1$, $T_2$, x und n gelten für die Verbinaungen der Formel IA dieselben Bevorzugungen wie für die Verbindungen der Formel I, ausgenommen der Ausführungen, die sich für n=1 auf $R_1$, $R_2$ und x beziehen.

Die Verbinaungen von Beispiel 10 ((N,N-Dibenzylaminoxymethyl)-N′-methyl-N′-2-(pyridin-2-yl)ethylamin) und Beispiel 12 ((N,N-Dibenzylaminoxymethyl)-N′-benzyl-N′-exo-norbornylamin) sind ebenfalls neu und können wie in den nachfolgenden Ausführungen beschrieben hergestellt werden.

Die Verbindungen der Formeln I und IA können in Analogie zu bekannten Verfahren hergestellt werden,

beispielsweise durch Umsetzung eines Hydroxylamins mit einer geeigneten Stickstoff-enthaltenden Verbindung und einem Aldehyd, bevorzugt Formaldehyd. Die Umsetzung wird vorzugsweise in einem Lösungsmittel, wie z.B. Acetonitril, Methylenchlorid oder Alkohol, insbesondere Methanol, durchgeführt.

Beispiele für Stickstoff-enthaltende Verbindungen sind Amine, insbesondere Di-n-butylamin, Di-n-hexylamin, Di-n-octylamin, Di-phenylamin, Diallylamin und Dibenzylamin, oder Diamine, bevorzugt 1,6-Hexamethylendiamin, oder heterocyclische Verbindungen, insbesondere Piperidin, Piperazin, Aminoalkylpyridin und Hexahydro-1,3,5-triazin, oder durch eine Aminogruppe substituiertes Norbornan.

Die Reaktionstemperatur liegt bevorzugt bei 0 bis 80°C.

Die Ausgangsverbindungen sind bekannt, teilweise im Handel erhältlich oder können in Analogie zu bekannten Verfahren hergestellt werden.

Die Verbindungen der Formeln I und IA eignen sich als Stabilisatoren für eine Reihe von organischen Materialien, wie beispielsweise Kunststoffe, Polymere und Harze, insbesondere

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha$,ß-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrol-polymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-tri-methylhexamethylenterephthalamid, Poly-m-phenylen-isophthalamid. Block-Copolymere der vorstehend genannten Polyamide mit Poly-olefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Poly-amidsysteme").

16. Polyharnstoffe, Polyimide, Polyamid-imide und Polybenzimid-azole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-di-methylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Cellulose-ether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Bevorzugt werden die Verbindungen der Formel I und IA in synthetischen Polymeren, insbesondere Polyolefinhomopolymeren oder -copolymeren, eingesetzt.

Besonders bevorzugt werden die Verbindungen der Formeln I und IA in Polyolefinen, wie beispielsweise Polyethylen und Polypropylen, Polystyrol, einschliesslich schlagfestem Polystyrol, Acrylnitril-Butadien-Styrol-Harz (ABS), Styrol-Butadien-Kautschuk (SBR), Isopren als auch natürlichem Kautschuk, Polyester, einschliesslich Polyethylenterephthalat und Polybutadienterephthalat sowie deren Copolymeren, und in Schmierölen, die beispielsweise aus Mineralölen hergestellt werden, eingesetzt.

Im allgemeinen können die Verbindungen der Formeln I und IA in Konzentrationen von ~0,01 bis ~5 Gew.-%, bezogen auf die stabilisierte Zusammensetzung, eingesetzt werden; allerdings kann je nach Verwendung und Substrat die Stabilisatormenge variieren. Bevorzugt verwendet man ~0,5 bis 2 Gew.-%, insbesondere 0,1 bis 1 Gew.-%.

Die Einarbeitung der Verbindungen der Formel I oder IA in das organische Material kann nach bekannten Methoden während jeder Verarbeitungsstufe vor der Formgebung erfolgen. Der Stabilisator kann beispielsweise in Pulverform dem organischen Material zugemischt werden oder eine Emulsion oder Suspension des Stabilisators kann mit einer Lösung, Suspension oder Emulsion des organischen Materials vermischt werden.

Die stabilisierten Zusammensetzungen dieser Erfindung können gegebenenfalls auch herkömmliche Additive enthalten.

Beispiele für herkömmliche Additive sind:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butyl-phenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2′-Thio-bis-(6-tert.butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

1.4. Alkyliden-Bisphenole, z.B. 2,2′-Methylen-bis-(6-tert.butyl-4-methylphenol), 2,2′-Methylen-bis-(6-tert.butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′0-Methylen-bis-(4,6-di-tert.butylphenol), 2,2′-Ethyliden-bis-(4,6-di-tert.butylphenol), 2,2′-Ethyliden-bis-(6-tert.butyl-4-isobutylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert.butylphenol), 4,4′-Methylen-bis-(6-tert.butyl-2-methylphenol), 1,1-Bis-(5-tert.butyl-4-hydroxy-2-methylphenyl)-butan, 2,6-Di-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3′-tert.butyl-4′-hydroxyphenyl)-butyrat], Di-(3-tert.butyl-4-hydroxy-5-methylphenyl)-dicyclopentadien, Di-[2-(3′-tert.butyl-2′-hydroxy-5′-methyl-benzyl)-6-tert.butyl-4-methyl-phenyl]-terephthalat.

1.5. Benzylverbindungen, z.B. 1,3,5-Tri-(3,5-di-tert.butyl-4-hydroxybenzyl)-2,4,6-trimethylbenzol, Di-(3,5-di-tert.butyl-4-hydroxybenzyl)-sulfid, 3,5-Di-tert.butyl-4-hydroxybenzyl-mercapto-essigsäure-isooctylester, Bis-(4-tert.butyl-3-hydroxy-2,6-dimethylbenzyl)dithiol-terephthalat,
1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxy-benzyl-phosphonsäure-dioctadecylester, 3,5-Di-tert.butyl-4-hydroxybenzyl-phosphonsäure-monoethylester, Calcium-salz.

1.6. Acylaminophenole, z.B. 4-Hydroxy-laurinsäureanilid, 4-Hydroxy-stearinsäureanilid, 2,4-Bis-octylmercapto-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-tert.butyl-4-hydroxyphenyl)-propionsäure mit ein-oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyl-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanat, Di-hydroxyethyl-oxalsäurediamid.

1.9. Amide der β-(3,5-Di-tert.butyl-4-hydroxyphenyl)-propionsäure, wie z.B. N,N′-Di-(3,5-di-tert.butyl-4-hydroxyphenylpropionyl)-hexamethylendiamin, N,N′-Di-(3,5-di-tert.butyl-4-hydroxyphenylpropionyl)-trimethylendiamin, N,N′-Di-(3,5-di-tert.butyl-4-hydroxyphenylpropionyl)-hydrazin.

2. UV-Absorber und Lichtschutzmittel

2.1. 2-(2′-Hydroxyphenyl)-benztriazole, wie z.B. das 5′-Methyl-, 3′,5′-Di-tert.butyl-, 5′-tert.Butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl, 4′-Octoxy-, 3′,5′-Di-tert.amyl-, 3′,5′-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Tri-hydroxy-, 2′-Hydroxy-4,4′-dimethoxy-Derivat.

2.3. Ester von gegebenenfalls substituierten Benzoesäuren, wie z.B. 4-tert.Butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyano-vinyl)-2-methyl-indolin.

2.5. Nickelverbindungen, wie z.B. Nickelkomplexe des 2,2′-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketonoxim, Nickelkomplexe des 1-Phenyl-4-lauroyl-5-hydroxypyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N′-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1′-(1,2-Ethandiyl)-bis-(3,3,5,5-tetramethylpiperazinon).

2.7. Oxalsäurediamide, wie z.B. 4,4′-Di-octyloxy-oxanilid, 2,2′-Dioctyloxy-5,5′-di-tert.butyl-oxanilid, 2,2′-Di-dodecyloxy-5,5′-di-tert.butyl-oxanilid, 2-Ethoxy-2′-ethyl-oxanilid, N,N′-Bis-(3-di-methylaminopropyl)-oxalamid, 2-Ethoxy-5-tert.butyl-2′-ethyloxanilid und dessen Gemisch mit 2-Ethoxy-2′-ethyl-5,4′-di-tert.butyl-oxanilid, Gemische von o-und p-Methoxy- sowie von o- und p-Ethoxy-di-substituierten Oxaniliden.

3. Metalldesaktivatoren, wie z.B. N,N′-Diphenyloxalsäurediamid, N-Salicylal-N′-salicyloylhydrazin, N,N′-Bis-salicyloylhydrazin, N,N′-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4′-biphenylen-diphosphonit, 3,9-Bis-(2,4-di-tert.butylphenoxy-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tri-decylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hyrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Bevorzugt enthalten die erfindungsgemässen Zusammensetzungen als weitere Zusätze ein Metallsalz einer höheren Fettsäure und/oder ein phenolisches Antioxidans.

Die Verbindungen der Formel I oder IA eignen sich sowohl allein als auch in Kombination mit anderen Additiven als Stabilisatoren für eine Reihe von Substraten, insbesondere Polyolefinen, die gegebenenfalls Alkalimetall-, Erdalkalimetall- oder Aluminiumsalze von höheren Fettsäuren (Beispiele sind die oben unter Punkt 7 aufgeführten Additive) und/oder gehinderte phenolische Antioxidantien enthalten können. Die Aminoxyalkylamine der Formeln I und IA vermindern sehr wirksam Verfärbungen, die auf die Anwesenheit von Phenolen zurückzuführen sind. Solche phenolischen Antioxidantien sind beispielsweise: n-Octadecyl-3,5-di-tert-butyl-4-hydroxyhydrocinnamat, Neopentantetrayl-tetrakis(3,5-di-tert-butyl-4-hydroxyhydrocinnamat), Di-n-octadecyl-3,5-di-tert-butyl-4-hydroxybenzylphosphonat, 1,3,5-Tris(3,5-di-tert-butyl-4-hydroxybenzyl)isocyanurat, Thiodiethylen-bis(3,5-di-tert-butyl-4-hydroxyhydrocinnamat), 1,3,5-Trimethyl-2,4,6-tris(3,5-di-tert-butyl-4-hydroxy- benzyl) benzol, 3,6-Dioxaoctamethylen-bis(3-methyl-5-tert-butyl-4-hydroxyhydrocinnamat), 2,6-Di-tert-butyl-p-cresol, 2,2′-Ethyliden-bis(4,6-di-tert-butylphenol), 1,3,5-Tris(2,6-dimethyl-4-tert-butyl-3-hydroxybenzyl)isocyanurat, 1,1,3-Tris(2-methyl-4-hydroxy-5-tert-butylphenyl)butan, 1,3,5-Tris[2-(3,5-di-tert-butyl-4-hydroxyhydrocinnamoyloxy)ethyl]isocyanurat, 3,5-Bis(3,5-di-tert-butyl-4-hydroxybenzyl)mesitol, Hexamethylen-bis(3,5-di-tert-butyl-4-hydroxyhydrocinnamat), 1-(3,5-Di-tert-butyl-4-hydroxyanilino)-3,5-bis(octylthio)-s-triazin, N,N′-Hexamethylen-bis(3,5-di-tert-butyl-4-hydroxyhydrocinnamamid), Kalzium-bis(ethyl-3,5-di-tert-butyl-4-hydroxybenzylphosphonat), Ethylen-bis[3,3-bis(3-tert-butyl-4-hydroxyphenyl)butyrat], Octyl-3,5-di-tert-butyl-4-hydroxybenzylmercaptoacetat, Bis(3,5-di-tert-butyl-4-hydroxyhydrocinnamoyl)hydrazid und N,N′-Bis[2-(3,5-di-tert-butyl-4-hydroxyhydrocinnamoyloxy)ethyl]oxamid, insbesondere Neopentantetrayl-tetrakis(3,5-di-tert- butyl-4-hydroxyhydrocinnamat), n-Octadecyl-3,5-di-tert-butyl-4-hydroxyhydrocinnamat, 1,3,5-Trimethyl-2,4,6-tris(3,5-di-tert-butyl-4-hydroxybenzyl)benzol, 1,3,5-Tris(3,5-di-tert-butyl-4-hydroxybenzyl)isocyanurat, 2,6-Di-tert-butyl-p-cresol oder 2,2′-Ethyliden-bis(4,6-di-tert-butylphenol).

Die Verbindungen der Formel I oder IA verhindern auch Verfärbungen, die auf die Anwesenheit von gehinderten Amin-Lichtstabilisatoren zurückzuführen sind, wie beispielsweise:
Bis(1,2,2,6,6-pentamethyl-4-piperidyl)-2-n-butyl-2-(3,5-di-tert-butyl-4-hydroxybenzyl)malonat, Bis(2,2,6,6-tetramethyl-4-piperidyl)sebacat, Umsetzungsprodukt von Dimethylsuccinat mit 1-Hydroxyethyl-2,2,6,6-tetramethyl-4-hydroxypiperidin und Umsetzungsprodukt von 2,4-Dichloro-6-octylamino-s-triazin mit N,N′-Bis(2,2,6,6-Tetramethyl-4-piperidyl)hexamethylendiamin.

Die folgenden Beispiele erläutern die Erfindung weiter. Alle Mengenangaben beziehen sich auf das Gewicht, soweit nicht anders angegeben.

Beispiel 1: Herstellung von (N,N-Dibenzylaminoxymethyl]piperidin (Verbindung 1)

Eine Lösung von 21,33 g Dibenzylhydroxylamin in 50 ml Acetonitril, welches 8,0 g einer 37%igen wässrigen Formaldehydlösung enthält, wird mit 8,7 g Piperidin gemischt. Die Reaktionslösung wird bei Raumtemperatur 24 Stunden gerührt. Das ausgefallene [N,N-Dibenzylaminoxymethyl]piperidin wird abfiltriert und mit Acetonitril umkristallisiert. Der Schmelzpunkt beträgt 57-60°C.

Elementaranalyse:

| | | | |
|---|---|---|---|
| Berechnet für $C_{20}H_{26}N_2O$: | C 77,4 %; | H 8,4 %; | N 9,0 % |
| Gefunden: | C 77,1 %; | H 8,4 % | N 9,0 % |

Beispiel 2: Herstellung von 1,4-Bis(N,N-Dibenzylaminoxymethyl]-piperazin (Verbindung 2)

Die Herstellung erfolgt in Analogie zu Beispiel 1. Es werden 21,33 g Dibenzylhyaroxylamin, 8,2 ml einer 37%igen wässrigen Formaldehydlösung und 4,31 g Piperazin eingesetzt. Nach Umkristallisation mit Acetonitril erhält man 21,8 g 1,4-Bis[N,N-Dibenzylaminoxymethyl]-piperazin, welches als weisser Feststoff vorliegt. Der Schmelzpunkt beträgt 132-135°C.

Elementaranalyse:

| | | | |
|---|---|---|---|
| Berechnet für $C_{34}H_{40}N_4O_2$: | C 76,1 %; | H 7,5 %; | N 10,4 % |
| Gefunden: | C 76,0 %; | H 7,7 % | N 10,5 % |

Beispiel 3: Herstellung von [N,N-Dibenzylaminoxymethyl]dimethylamin (Verbingung 3)

Eine Mischung von 4,86 g Dibenzylhydroxylamin, 2,42 g wasserfreiem Natriumcarbonat und 4,22 g N,N-Dimethyl-methyleniminiumjodid in 50 ml Methylenchlorid wird 48 Stunden bei Raumtemperatur unter Stickstoff gerührt. Anschliessend wird der unlösliche anorganische Rückstand abfiltriert und die Lösung bei vermindertem Druck eingeengt. Der erhaltene Rückstand wird mit Heptan extrahiert. Die Heptan-Extrakte werden verworfen. Man erhält das [N,N-Dibenzylaminoxymethyl]dimethylamin als dickes Oel.

Elementaranalyse:

| | | | |
|---|---|---|---|
| Berechnet für $C_{17}H_{22}N_2O$: | C 75,5 %; | H 8,2 %; | N 10,4 % |
| Gefunden: | C 75,4 %; | H 8,2 % | N 9,8 % |

Beispiel 4: Herstellung von N,N'-Dimethyl-N,N'-bis(dibenzylaminoxymethyl)]hexamethylendiamin (Verbindung 4)

Die Herstellung erfolgt in Analogie zu Beispiel 1. Es werden 21,33 g Dibenzylhydroxylamin, 8,2 ml einer 37%igen wässrigen Formaldehydlösung und 7,36 g N,N'-Dimethyl-1,6-hexamethylendiamin in 100 ml Acetonitril eingesetzt. Man erhält 26,6 g N,N'-Dimethyl-N,N'-bis-(dibenzylaminoxymethyl)]hexamethylendiamin, welches als farblose Flüssigkeit vorliegt.

Elementaranalyse:

| | | | |
|---|---|---|---|
| Berechnet für $C_{38}H_{50}N_4O_2$: | C 76,7 %; | H 8,5 %; | N 9,4 % |
| Gefunden: | C 76,1 %; | H 8,5 % | N 9,4 % |

Beispiel 5: Herstellung von [N,N-Dibenzylaminoxymethyl]di-n-octylamin (Verbingung 5)

Die Herstellung erfolgt in Analogie zu Beispiel 1. Es werden 21,33 g Dibenzylhydroxylamin, 9,73 g einer wässrigen Formaldehydlösung und 24,15 g Di-n-octylamin in Acetonitril eingesetzt.

Elementaranalyse:

| | | | |
|---|---|---|---|
| Berechnet für $C_{33}H_{50}N_2O$: | C 79,8 %; | H 10,8 %; | N 6,0 % |
| Gefunden: | C 79,6 %; | H 10,6 % | N 5,9 % |

Beispiel 6: Herstellung von [N,N-Dibenzylaminoxymethyl]di-n-butylamin (Verbindung 6)

Die Herstellung erfolgt in Analogie zu Beispiel 1. Es werden 21,33 g Dibenzylhyaroxylamin, 9,73 g einer 37%igen wässrigen Formaldehydlösung und 12,93 g Di-n-butylamin in Acetonitril eingesetzt.

Elementaranalyse:

| | | | |
|---|---|---|---|
| Berechnet für $C_{23}H_{34}N_2O$: | C 77,9 %; | H 9,7 %; | N 7,9 % |
| Gefunden: | C 78,2 %; | H 9,4 % | N 7,9 % |

Beispiel 7: Herstellung von [N,N-Dibenzylaminoxymethyl)di-n-hexylamin (Verbindung 7)

Die Herstellung erfolgt in Analogie zu Beispiel 1. Es werden 21,33 g Dibenzylhydroxylamin, 9,73 g einer 37%igen wässrigen Formaldehydlösung und 18,54 g Di-n-hexylamin eingesetzt.

Elementaranalyse:

| | | | |
|---|---|---|---|
| Berechnet für $C_{27}H_{42}N_2O$: | C 79,0 %; | H 10,3 %; | N 6,8 % |
| Gefunden: | C 79,2 %; | H 10,3 % | N 6,8 % |

Beispiel 8: Herstellung von [N,N-Dibenzylaminoxymethyl]diisopropylamin (Verbindung 8)

Die Herstellung erfolgt in Analogie zu Beispiel 1. Es werden 21,33 g Dibenzylhydroxylamin, 9,73 g einer 37%igen wässrigen Formaldehydlösung und 10,12 g Diisopropylamin eingesetzt.

Elementaranalyse:

| | | | |
|---|---|---|---|
| Berechnet für $C_{21}H_{30}N_2O$: | C 77,3 %; | H 9,3 %; | N 8,6 % |
| Gefunden: | C 77,6 %; | H 9,3 % | N 8,4 % |

Beispiel 9: Herstellung von [N,N-Dibenzylaminoxymethyl]diallylamin (Verbingung 9)

Die Herstellung erfolgt in Analogie zu Beispiel 1. Es werden 21,33 g Dibenzylhydroxylamin, 9,73 g einer 37%igen wässrigen Formaldehydlösung und 9,72 g Diallylamin eingesetzt.

Elementaranalyse:

| | | | |
|---|---|---|---|
| Berechnet für $C_{21}H_{26}N_2O$: | C 78,2 %; | H 8,1 %; | N 8,7 % |
| Gefunden: | C 78,1 %; | H 8,2 % | N 8,6 % |

Beispiel 10: Herstellung von [N,N-Dibenzylaminoxymethyl]-N′-methyl-N′-2-(pyridin-2-yl)ethylamin (Verbindung 10)

Die Herstellung erfolgt in Analogie zu Beispiel 1. Es werden 21,33 g Dibenzylhydroxylamin, 9,73 g einer 37%igen wässrigen Formaldehydlösung und 13,62 g 2-[2-(N-Methylamino)ethyl]pyridin eingesetzt.

Elementaranalyse:

| | | | |
|---|---|---|---|
| Berechnet für $C_{23}H_{27}N_3O$: | C 76,4 %; | H 7,5 %; | N 11,6 % |
| Gefunden: | C 76,0 %; | H 7,4 % | N 11,5 % |

Beispiel 11: Herstellung von [N,N-Dibenzylaminoxymethyl]diphenylamin (Verbindung 11)

Die Herstellung erfolgt in Analogie zu Beispiel 1. Es werden 21,33 g Dibenzylhydroxylamin, 9,46 g einer 37%igen wässrigen Formaldehydlösung und 16,92 g Diphenylamin eingesetzt. [N,N-Dibenzylaminoxymethyl]diphenylamin liegt als farblose Flüssigkeit vor. Das Massenspektrum sowie die NMR- und IR-Spektren stimmen mit der gewünschten Struktur überein.

Beispiel 12: Herstellung von (N,N-Dibenzylaminoxymethyl]-N′-benzyl-N′-(exo-norbornyl)amin (Verbindung 12)

Die Herstellung erfolgt in Analogie zu Beispiel 1. Es werden 21,33 g Dibenzylhydroxylamin, 9,73 g einer 37%igen wässrigen Formaldehydlösung und 20,13 g 2-(Benzylamino)norbornan eingesetzt.

Elementaranalyse:

| | | | |
|---|---|---|---|
| Berechnet für $C_{27}H_{34}N_2O$: | C 81,7 %; | H 8,0 %; | N 6,6 % |
| Gefunden: | C 81,6 %; | H 7,7 % | N 6,5 % |

Beispiel 13: Herstellung von N′,N″,N‴-Tris[N,N-Dibenzylaminoxymethyl]hexahydro-s-triazin (Verbindung 13)

Die Herstellung erfolgt in Analogie zu Beispiel 1. Es werden 21,33 g Dibenzylhydroxylamin, 16,0 g einer 37%igen wässrigen Formaldehydlösung und 2,90 g Hexahydro-s-triazin eingesetzt.

Beispiel 14: Herstellung von [N,N-Diethylaminoxymethyl]di-n-butylamin (Verbindung 14)

Die Herstellung erfolgt in Analogie zu Beispiel 1. Es werden 26,74 g Diethylhyaroxylamin, 29,22 g einer 37%igen wässrigen Formaldehydlösung und 38,77 g Di-n-butylamin eingesetzt. [N,N-Diethylaminoxymethyl]di-n-butylamin liegt als farblose Flüssigkeit vor.

Elementaranalyse:

| | | | |
|---|---|---|---|
| Berechnet für $C_{13}H_{30}N_2O$: | C 67,8 %; | H 13,1 %; | N 12,2 % |
| Gefunden: | C 67,6 %; | H 13,5 %; | N 11,8 % |

Beispiel 15: Herstellung von [N,N-Diethylaminoxymethyl]diallylamin (Verbindung 15)

Die Herstellung erfolgt in Analogie zu Beispiel 1. Es werden 26,74 g Diethylhydroxylamin, 10,81 g einer 37%igen wässrigen Formaldehydlösung und 29,15 g Diallylamin eingesetzt.

Elementaranalyse:

| | | | |
|---|---|---|---|
| Berechnet für $C_{11}H_{22}N_2O$: | C 66,6 %; | H 11,2 %; | N 14,1 % |
| Gefunden: | C 66,7 %; | H 10,8 % | N 14,0 % |

Beispiel 16: Verarbeitungsstabilität von Polypropylen

Die Grundformulierung enthält 100 Teile unstabilisiertes Polypropylen (®Profax 6501, Himont) und 0,1 Teil Kalziumstearat. Die unten angegebenen Stabilisatoren werden in Form einer Lösung dem Polypropylen zugemischt (Lösungsmittel: Methylenchlorid). Das Lösungsmittel wird im Vakuum entfernt. Die stabilisierte Harzformulierung wird bei 100 Umdrehungen pro Minute extrudiert.

Extrusionsbedindungen

| | |
|---|---|
| Zylinder 1 | 232°C |
| Zylinder 2 | 246°C |
| Zylinder 3 | 260°C |
| Düse 1 | 260°C |
| Düse 2 | 260°C |
| Düse 3 | 260°C |

Nach der ersten, dritten und fünften Extrusion werden die HarzKügelchen unter Druck bei 193°C zu 3,2 mm dicken Folien verformt.

Der "Yellowness Index" der Proben wird gemäss ASTM D 1925-63 T bestimmt. Niedrige Werte bedeuten eine geringe Verfärbung.

Der Schmelzindex der Proben wird gemäss ASTM 1238-L bestimmt. Höhere Werte bedeuten eine geringere Molmasse und sind ein Hinweis dafür, dass das Polymer abgebaut ist.

Die Ergebnisse der verschiedenen Testserien sind in den folgenden Tabellen aufgeführt.

Serie 1:

| Stabilisator | Extrusionstemperatur 260°C "Yellowness Index" nach der 1. Extrusion | 3. Extrusion | 5. Extrusion |
|---|---|---|---|
| ohne | 2,5 | 3,5 | 4,7 |
| 0,1 % Antioxidans A | 7,4 | 13,2 | 15,9 |
| 0,1 % Antioxidans A plus 0,5 % Verbindung 2 | 6,2 | 7,6 | 8,5 |
| 0,1 % Antioxidans A plus 0,05 % Verbindung 4 | 4,7 | 6,0 | 6,7 |
| 0,1 % Antioxidans A plus 0,05 % Verbindung 7 | 3,2 | 3,7 | 3,9 |
| 0,1 % Antioxidans A plus 0,05 % Verbindung 8 | 1,3 | 1,9 | 2,4 |

| Stabilisator | "Schmelzindex" in g/10 min nach der 1. Extrusion | 5. Extrusion |
|---|---|---|
| ohne | 6,3 | 14,9 |
| 0,1 % Antioxidans A | 3,4 | 6,9 |
| 0,1 % Antioxidans A plus 0,5 % Verbindung 2 | 3,0 | 3,5 |
| 0,1 % Antioxidans A plus 0,05 % Verbindung 4 | 3,4 | 4,2 |
| 0,1 % Antioxidans A plus 0,05 % Verbindung 7 | 3,2 | 4,3 |
| 0,1 % Antioxidans A plus 0,05 % Verbindung 8 | 3,2 | 3,9 |

Serie 2:

| Stabilisator | Extrusionstemperatur 260°C<br>"Yellowness Index" nach der<br>1. Extrusion | 3. Extrusion | 5. Extrusion |
|---|---|---|---|
| ohne | 1,8 | 2,5 | 3,5 |
| 0,1 % Antioxidans A | 4,6 | 8,9 | 10,9 |
| 0,1 % Antioxidans A plus 0,05 % Verbindung 11 | 2,6 | 3,6 | 6,2 |

| Stabilisator | "Schmelzindex" in g/10 min nach der<br>1. Extrusion | 5. Extrusion |
|---|---|---|
| ohne | 5,0 | 9,9 |
| 0,1 % Antioxidans A | 2,8 | 4,8 |
| 0,1 % Antioxidans A plus 0,05 % Verbindung 11 | 2,4 | 3,4 |

Serie 3:

| Stabilisator | Extrusionstemperatur 260°C "Yellowness Index" nach der 1. Extrusion | 3. Extrusion | 5. Extrusion |
|---|---|---|---|
| ohne | 1,6 | 1,1 | 1,3 |
| 0,1 % Antioxidans A | 6,0 | 11,7 | 14,6 |
| 0,1 % Antioxidans A plus 0,05 % Verbindung 6 | 2,4 | 3,4 | 6,5 |
| 0,1 % Antioxidans A plus 0,05 % Verbindung 14 | 5,3 | 8,2 | 10,8 |

| Stabilisator | "Schmelzindex" in g/10 min nach der 1. Extrusion | 5. Extrusion |
|---|---|---|
| ohne | 1,3 | 12,5 |
| 0,1 % Antioxidans A | 4,0 | 5,9 |
| 0,1 % Antioxidans A plus 0,05 % Verbindung 6 | 2,4 | 4,4 |
| 0,1 % Antioxidans A plus 0,05 % Verbindung 14 | 3,0 | 3,4 |

Antioxidans A: Neopentyl-tetrakis[3-(3',5'-di-tert-butyl-4'-hydroxyphenyl)propanoat]

Beispiel 17: Lichtstabilität von Polypropylen

Unstabilisiertes Polypropylenpulver (®Hercules Profax 6501) wird mit dem in Tabelle 1 angegebenen Additiv gemischt. Diese Mischung wird bei 182°C 5 Minuten auf einem Mischwalzwerk plastifiziert. Dann löst man die stabilisierte Polypropylenfolie vom Walzwerk ab und lässt sie abkühlen. Die Folie wird in Stücke geschnitten, welche mit einer hydraulischen Presse bei 250°C und 12 bar zu 0,635 mm dicken Filmen verformt werden.

Die Proben werden in einer "fluorescent sunlight/black light chamber" (FS/BL) belichtet. Der Carbonylgehalt der Prüflinge wird mit IR-Spektroscopie bestimmt. Als Mass für die Wirksamkeit des Stabilisators gilt die Zeit bis zum Erreichen einer Carbonylabsorption von 0,5. Die Ergebnisse sind In Tabelle 1 aufgeführt.

Tabelle 1:

| Stabilisator | Stunden bis zum Erreichen einer Carbonylabsorption von 0,5 |
|---|---|
| ohne | 100 |
| 0,2 % Verbindung 1 | 320 |
| 0,2 % Verbindung 2 | 320 |
| 0,2 % Verbindung 3 | 290 |
| 0,2 % Verbindung 4 | 310 |
| 0,2 % Verbindung 5 | 310 |
| 0,2 % Verbindung 6 | 200 |
| 0,2 % Verbindung 8 | 280 |
| 0,2 % Verbindung 9 | 290 |
| 0,2 % Verbindung 10 | 290 |
| 0,2 % Verbindung 12 | 270 |
| 0,2 % Verbindung 14 | 230 |

Beispiel 18: Verbinderung der Oxidation von Petroleum-Turbinenöl

Der Test wird gemäss ASTM D 943-81 durchgeführt. 300 ml 150 N paraffinisches Mineralöl, welches 0,25 Gew.% der zu prüfenden Verbindung enthält, und 60 ml destilliertes Wasser werden in eine grosse Glasröhre gefüllt und in einem 95°C heissem Oelbad erhitzt. In das Oel-Wasser-Gemisch wird Sauerstoff (3 l/h) eingeleitet. Als Katalysator befindet sich im Gaseinleitungsrohr eine Eisen-Kupfer-Spirale. In regelmässigen Zeitabständen werden Proben des Oels entnommen und die Säurezahl bestimmt. Als kriterium für die Stabilisierung des Oels gilt die Zeit bis zum Erreichen einer Säurezahl von 2,0. Die Versuchsergebnisse werden in Tabelle 2 wiedergegeben.

Tabelle 2:

| Stabilisator | Stunden bis zum Erreichen einer Säurezahl von 2,0 |
|---|---|
| ohne | 90-100 |
| Verbindung 4 | 295 |

Beispiel 19: TFOUT-Test (Thin-Film Oxygen Uptake Test)

Der Test wird in einer Standardapparatur gemäss ASTM D-2272 durchgeführt. Die Modifikationen in der Durchführung werden in der Kopie Nr. 82 GC-10-1 von der Tagung "Conference of the American Society of Lubrication Engineers, October 5-7, 1982" beschrieben.

Eine Probe (1,5 g) eines 150 N paraffinischen Mineralöls,welches 0,5 Geh.% der zu testenden Verbindung und soviel Zinkdialkyldithiophosphat enthält, wie 0,1 Gew.% Zink entspricht, wird in die Versuchsapparatur gegeben. Eine Katalysatormischung, die 0,075 g von oxidierten Treibstoffkomponenten, 0,075 g eines löslichen Metallkatalysators*) und 0,030 g Wasser enthält, wird hinzugefügt. Die Temperatur wird auf 160°C eingestellt und der anfängliche Sauerstoffdruck beträgt 6,2 bar. Als Kriterium für die Stabilisierung des Mineralöls gilt die Zeit in Minuten bis zu einem Druckabfall von 1,72 bar. Die Versuchsergebnisse werden in Tabelle 3 wiedergegeben.

Tabelle 3:

| Stabilisator | Zeit in Minuten bis zu einem Druckabfall von 1,72 bar |
|---|---|
| ohne | 105 |
| Verbindung 4 | 125 |

*) Bei dem löslichen Metallkatalysator handelt es sich um ein Gemisch aus Metallnaphthenaten (0,69 Gew.% Kupfer-, 0,41 Gew.% Eisen-, 8,0 Gew.% Blei-, 0,35 Gew.% Mangan- und 0,36 Gew.% Zinnaphthenat.

## Patentansprüche

1. Zusammensetzungen enthaltend ein gegen oxidativen, thermischen und/oder aktinischen Abbau empfindliches organisches Material und mindestens eine Verbindung der Formel I,

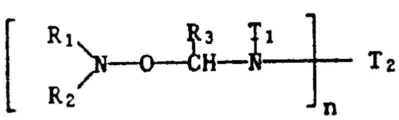

(I)

worin $R_1$ und $R_2$ unabhängig voneinander $C_1$-$C_{36}$-Alkyl, $C_5$-$C_{12}$-Cycloalkyl, $C_7$-$C_9$-Aralkyl oder durch $C_1$-$C_{36}$-Alkyl substituiertes $C_7$-$C_9$-Aralkyl bedeuten oder $R_1$ und $R_2$ zusammen mit dem Stickstoffatom, an das sie gebunden sind, einen 5- bis 7-gliedrigen heterocyclischen Ring bilden oder $R_1$ und $R_2$ unabhängig voneinander eine Gruppe der Formel (II)

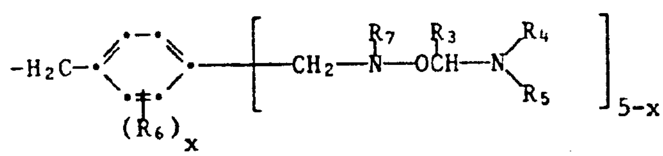

(II)

sind, $R_3$, $R_4$, $R_5$, $R_6$ und $R_7$ unabhängig voneinander Wasserstoff, $C_1$-$C_{36}$-Alkyl, $C_5$-$C_{12}$-Cycloalkyl, Allyl, Aryl, $C_7$-$C_9$-Aralkyl, durch $C_1$-$C_{36}$-Alkyl substituiertes $C_7$-$C_9$-Aralkyl, Bornyl oder Norbornyl darstellen oder $R_4$ und $R_5$ zusammen mit dem Stickstoffatom, an das sie gebunden sind, einen 5- bis 7-gliedrigen heterocyclischen Ring bilden, x eine ganze Zahl von 0 bis 5 und n von 1 bis 4 bedeuten, $T_1$ eine der für $R_3$ angegebenen Bedeutungen besitzt oder

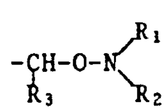

ist,

wenn n 1 ist, $T_2$ eine der für $R_3$ angegebenen Bedeutungen hat oder $C_1$-$C_4$-Alkyl, welches durch einen 5- bis 7-gliedrigen, ungesättigten heterocyclischen Ring substituiert ist, bedeutet oder die Reste $T_1$ und $T_2$ zusammen mit dem Stickstoffatom, an das sie gebunden sind, einen 5- bis 7-gliedrigen, gesättigten heterocyclischen Ring bilden,

wenn n 2 bedeutet, $T_2$ $C_2$-$C_{12}$-Alkylen, $C_6$-$C_{10}$-Cycloalkylen, $C_6$-$C_{10}$-Arylen oder $C_8$-$C_{10}$-Alkylenarylenalkylen ist oder $T_1$ und $T_2$ zusammen mit den beiden Stickstoffatomen, an die sie gebunden sind, einen 5- bis 7-gliedrigen, gesättigten heterocyclischen Ring bilden,

wenn n 3 bedeutet, $T_2$ $C_3$-$C_6$-Alkantriyl ist oder $T_1$ und $T_2$ zusammen mit den drei Stickstoffatomen, an die sie gebunden sind, einen 5-bis 6-gliedrigen, gesättigten heterocyclischen Ring bilden und

wenn n 4 bedeutet, $T_2$ $C_4$-$C_6$-Alkantetrayl darstellt.

2. Zusammensetzung gemäss Anspruch 1, worin die Reste $R_1$ bis $R_7$ unabhängig voneinander $C_1$-$C_{18}$-Alkyl, Cyclopentyl, Cyclohexyl, Benzyl, α-Methylbenzyl oder α,α-Dimethylbenzyl sind und $R_3$ zusätzlich Wasserstoff bedeutet.

3. Zusammensetzung gemäss Anspruch 1, worin $R_1$ und $R_2$ Benzyl sind und $R_3$ Wasserstoff bedeutet.

4. Zusammensetzung gemäss Anspruch 1, worin n 1 ist, $T_1$ und $T_2$ unabhängig voneinander $C_1$-$C_{18}$-Alkyl, Cyclopentyl, Cyclohexyl, Allyl, Phenyl, Benzyl, α-Methylbenzyl oder α,α-Dimethylbenzyl bedeuten oder die Reste $T_1$ und $T_2$ zusammen mit dem Stickstoffatom, an das sie gebunden sind, eine Morpholino-, Pyrrolidino- oder Piperidinogruppe bilden.

5. Zusammensetzung gemäss Anspruch 1, worin n 2 bedeutet, $T_2$ $C_2$-$C_6$-Alkylen, Phenylen oder Xylylen ist oder $T_1$ und $T_2$ zusammen mit den beiden Stickstoffatomen, an die sie gebunden sind, einen Piperazinrest bilden.

6. Zusammensetzung gemäss Anspruch 1, worin n 3 bedeutet, $T_2$ Glyceryl oder Trimethylylpropan ist oder $T_1$ und $T_2$ zusammen mit den drei Stickstoffatomen, an die sie gebunden sind, einen Hexahydro-1,3,5-triazinring bilden.

7. Zusammensetzung gemäss Anspruch 1, worin n 4 bedeutet und $T_2$ Pentaerythrityl ist.

8. Zusammensetzung gemäss Anspruch 1, worin $R_1$ und $R_2$ unabhängig voneinander $C_1$-$C_6$-Alkyl oder Benzyl sind, $R_3$ Wasserstoff ist, n 1, 2 oder 3 bedeutet und wenn n 1 ist, $T_1$ und $T_2$ unabhängig voneinander $C_1$-$C_8$-Alkyl, Allyl, Phenyl, Benzyl oder Norbornyl sind und $T_2$ zusätzlich $C_1$-$C_4$-Alkyl, welches durch eine Pyridinylgruppe substituiert ist, bedeutet oder $T_1$ und $T_2$ zusammen mit dem Stickstoffatom, an das sie gebunden sind, eine Piperidinogruppe bilden, wenn n 2 ist, $T_1$ $C_1$-$C_6$-Alkyl und $T_2$ $C_2$-$C_6$-Alkylen sind oder $T_1$ und $T_2$ zusammen mit den beiden Stickstoffatomen, an die sie gebunden sind, einen Piperazinring bilden und wenn n 3 bedeutet, $T_1$ und $T_2$ zusammen mit den drei Stickstoffatomen, an die sie gebunden sind, eine Hexahydro-1,3,5-triazingruppe bilden.

9. Zusammensetzung gemäss Anspruch 1, worin die Verbindung der Formel I
1,4-Bis[N,N-dibenzylaminoxymethyl]piperazin,
N′,N″-Dimethyl-N′,N″-bis[N,N-dibenzylaminoxymethyl]hexamethylendiamin,
[N,N-Dibenzylaminoxymethyl]di-n-butylamin,
[N,N-Dibenzylaminoxymethyl]di-n-hexylamin,
[N,N-Dibenzylaminoxymethyl]diisopropylamin,
[N,N-Diethylaminoxymethyl]di-n-butylamin,
[N,N-Dibenzylaminoxymethyl]-N′-methyl-N′-2-(pyridin-2-yl)ethylamin,
[N,N-Dibenzylaminoxymethyl]-N′-benzyl-N′-exo-norbornylamin oder
N′,N″,N‴-Tris[N,N-dibenzylaminoxymethyl]hexahydro-1,3,5-triazin ist.

10. Zusammensetzung gemäss Anspruch 1, worin das organische Material ein synthetisches Polymer ist.

11. Zusammensetzung gemäss Anspruch 10, worin das synthetische Polymer ein Polyolefinhomopolymer oder -copolymer ist.

12. Zusammensetzung gemäss Anspruch 1, die zusätzlich ein Metallsalz einer höheren Fettsäure und/oder ein phenolisches Antioxidans enthält.

13. Zusammensetzung gemäss Anspruch 12, worin des phenolische Antioxidans Neopentantetrayl-tetrakis(3,5-di-tert-butyl-4-hydroxyhydrocinnamat), n-Octadecyl-3,5-di-tert-butyl-4-hydroxyhydrocinnamat, 1,3,5-Trimethyl-2,4,6-tris(3,5-di-tert-butyl-4-hydroxybenzyl)-benzol,
1,3,5-Tris(3,5-di-tert-butyl-4-hydroxybenzyl)isocyanurat, 2,6-Di-tert-butyl-p-cresol oder 2,2′-Ethyliden-bis(4,6-di-tert-butylphenol) ist.

14. Verwendung von Verbindungen der Formel I gemäss Anspruch 1 zum Stabilisieren von organischem Material gegen thermischen, oxidativen und/oder aktinischen Abbau.

15. Verbindungen der Formel IA,

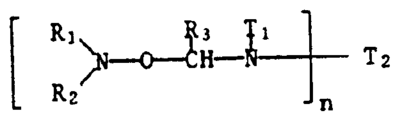

(IA)

worin n eine ganze Zahl von 1 bis 4 bedeutet, wenn n 1 ist, $R_1$ und $R_2$ unabhängig voneinander eine Gruppe der Formel (IIA)

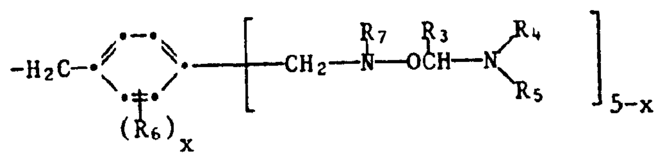

(IIA)

sind, $R_3$, $R_4$, $R_5$, $R_6$ und $R_7$ unabhängig voneinander Wasserstoff, $C_1$-$C_{36}$-Alkyl, $C_5$-$C_{12}$-Cycloalkyl, Allyl, Aryl, $C_7$-$C_9$-Aralkyl, durch $C_1$-$C_{36}$-Alkyl substituiertes $C_7$-$C_9$-Aralkyl, Bornyl oder Norbornyl darstellen oder $R_4$ und $R_5$ zusammen mit dem Stickstoffatom, an das sie gebunden sind, einen 5- bis 7-gliedrigen heterocyclischen Ring bilden, x eine ganze Zahl von 0 bis 4 bedeutet, $T_1$ eine der für $R_3$ angegebenen Bedeutungen besitzt oder

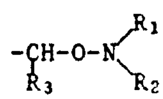

ist,

$T_2$ eine der für $R_3$ angegebenen Bedeutungen hat oder $C_1$-$C_4$-Alkyl, welches durch einen 5- bis 7-gliedrigen, ungesättigten heterocyclischen Ring substituiert ist, bedeutet oder die Reste $T_1$ und $T_2$ zusammen mit dem Stickstoffatom, an das sie gebunden sind, einen 5- bis 7-gliedrigen, gesättigten heterocyclischen Ring bilden, wenn n 2, 3 oder 4 bedeutet, $R_1$ und $R_2$ unabhängig voneinander $C_1$-$C_{36}$-Alkyl, $C_5$-$C_{12}$-Cycloalkyl, $C_7$-$C_9$-Aralkyl oder durch $C_1$-$C_{36}$-Alkyl substituiertes $C_7$-$C_9$-Aralkyl bedeuten oder $R_1$ und $R_2$ zusammen mit dem Stickstoffatom, an das sie gebunden sind, einen 5- bis 7-gliedrigen heterocyclischen Ring bilden oder $R_1$ und $R_2$ unabhängig voneinander eine Gruppe der Formel (II)

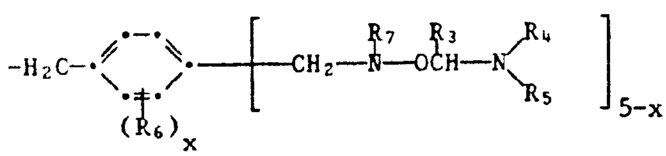

(II)

sind, $R_3$, $R_4$, $R_5$, $R_6$ und $R_7$ unabhängig voneinander Wasserstoff, $C_1$-$C_{36}$-Alkyl, $C_5$-$C_{12}$-Cycloalkyl, Allyl, Aryl, $C_7$-$C_9$-Aralkyl, durch $C_1$-$C_{36}$-Alkyl substituiertes $C_7$-$C_9$-Aralkyl, Bornyl oder Norbornyl darstellen oder $R_4$ und $R_5$ zusammen mit dem Stickstoffatom, an das sie gebunden sind, einen 5- bis 7-gliedrigen heterocyclischen Ring bilden, x eine ganze Zahl von 0 bis 5 bedeutet, $T_1$ eine der für $R_3$ angegebenen Bedeutungen besitzt oder

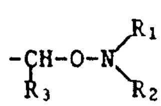

ist,

wenn n 2 bedeutet, $T_2$ $C_2$-$C_{12}$-Alkylen, $C_6$-$C_{10}$-Cycloalkylen, $C_6$-$C_{10}$-Arylen oder $C_8$-$C_{10}$-Alkylenarylenalkylen ist oder $T_1$ und $T_2$ zusammen mit den beiden Stickstoffatomen, an die sie gebunden sind, einen 5- bis 7-gliedrigen, gesättigten heterocyclischen Ring bilden,

wenn n 3 bedeutet, $T_2$ $C_3$-$C_6$-Alkantriyl ist oder $T_1$ und $T_2$ zusammen mit den drei Stickstoffatomen, an die sie gebunden sind, einen 5- bis 6-gliedrigen, gesättigten heterocyclischen Ring bilden und

wenn n 4 bedeutet, $T_2$ $C_4$-$C_6$-Alkentetrayl darstellt.

16. Die Verbindungen (N,N-Dibenzylaminoxymethyl)-N′-methyl-N′-2-(pyridin-2-yl)ethylamin und (N,N-Dibenzylaminoxymethyl)-N′-benzyl-N′-exo-norbornylamin.

## Claims

1. A composition comprising an organic material sensitive to oxidative, thermal and/or actinic degradation and at least one compound of the formula I

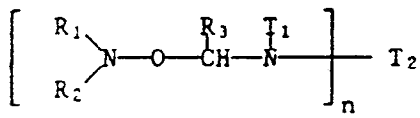

(I)

in which $R_1$ and $R_2$ are independently of one another $C_1$-$C_{36}$alkyl, $C_5$-$C_{12}$cycloalkyl, $C_7$-$C_9$aralkyl or $C_7$-$C_9$aralkyl substituted by $C_1$-$C_{36}$alkyl or $R_1$ and $R_2$ together with the nitrogen atom to which they are bonded form a 5- to 7- membered heterocyclic ring, or $R_1$ and $R_2$ are independently of one another a group of the formula (II)

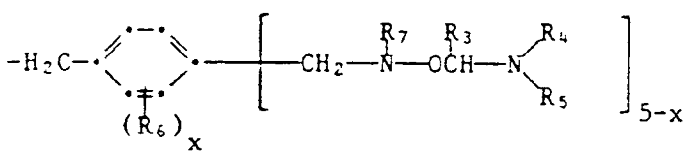

(II);

$R_3$, $R_4$, $R_5$, $R_6$ and $R_7$ are independently of one another hydrogen, $C_1$-$C_{36}$alkyl, $C_5$-$C_{12}$cycloalkyl, allyl, aryl, $C_7$-$C_9$aralkyl, $C_7$-$C_9$aralkyl substituted by $C_1$-$C_{36}$alkyl, bornyl or norbomyl, or $R_4$ and $R_5$ together with the nitrogen atom to which they are bonded form a 5- to 7- membered heterocyclic ring, x is an integer from 0 to 5 and n is 1 to 4, $T_1$ has one of the meanings given for $R_3$ or is

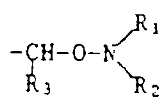

,

when n is 1, $T_2$ has one of the meanings given for $R_3$ or is $C_1$-$C_4$alkyl which is substituted by a 5- to 7- membered unsaturated heterocyclic ring, or the radicals $T_1$ and $T_2$ together with the nitrogen atom to which they are bonded form a 5- to 7- membered saturated heterocyclic ring,

when n is 2, $T_2$ is $C_2$-$C_{12}$alkylene, $C_6$-$C_{10}$cycloalkylene, $C_6$-$C_{10}$arylene or $C_8$-$C_{10}$alkylenearylenealkylene or $T_1$ and $T_2$ together with the two nitrogen atoms to which they are bonded form a 5- to 7- membered saturated heterocyclic ring,

when n is 3, $T_2$ is $C_3$-$C_6$alkanetriyl or $T_1$ and $T_2$ together with the three nitrogen atoms to which they are bonded form a 5- to 6- membered saturated heterocyclic ring and

when n is 4, $T_2$ is $C_4$-$C_6$alkanetetrayl.

2. A composition according to claim 1, in which the radicals $R_1$ to $R_7$ are independently of one another $C_1$-$C_{18}$alkyl, cyclopentyl, cyclohexyl, benzyl, α-methylbenzyl or α,α-dimethylbenzyl and $R_3$ is additionally hydrogen.

3. A composition according to claim 1, in which $R_1$ and $R_2$ are benzyl and $R_3$ is hydrogen.

4. A composition according to claim 1, in which n is 1, $T_1$ and $T_2$ are independently of one another $C_1$-$C_{18}$alkyl, cyclopentyl, cyclohexyl, allyl, phenyl, benzyl, α-methylbenzyl or α,α-dimethylbenzyl or the radicals $T_1$ and $T_2$ together with the nitrogen atom to which they are bonded, form a morpholino, pyrrolidino or piperidino group.

5. A composition according to claim 1, in which n is 2, $T_2$ is $C_2$-$C_6$alkylene, phenylene or xylylene, or $T_1$ and $T_2$ together with the two nitrogen atoms to which they are bonded form a piperazine radical.

6. A composition according to claim 1, in which n is 3, $T_2$ is glyceryl or trimethylylpropane, or $T_1$ and $T_2$ together with the three nitrogen atoms to which they are bonded form a hexahydro-1,3,5-triazine ring.

7. A composition according to claim 1, in which n is 4 and $T_2$ is pentaerythrityl.

8. A composition according to claim 1, in which $R_1$ and $R_2$ are independently of one another $C_1$-$C_6$alkyl or benzyl, $R_3$ is hydrogen, n is 1, 2 or 3, and when n is 1, $T_1$ and $T_2$ are independently of one another $C_1$-$C_8$alkyl,

allyl, phenyl, benzyl or norbornyl, and $T_2$ is additionally $C_1$-$C_4$alkyl which is substituted by a pyridinyl group, or $T_1$ and $T_2$ together with the nitrogen atom to which they are bonded form a piperidino group, when n is 2, $T_1$ is $C_1$-$C_6$alkyl and $T_2$ is $C_2$-$C_6$alkylene, or $T_1$ and $T_2$ together with the two nitrogen atoms to which they are bonded form a piperazine ring, and when n is 3, $T_1$ and $T_2$ together with the three nitrogen atoms to which they are bonded form a hexahydro-1,3,5-triazine group.

9. A composition according to claim 1, in which the compound of the formula I is
1,4-bis[N,N-dibenzylaminoxymethyl]piperazine,
N',N''-dimethyl,N',N''-bis[N,N-dibenzylaminoxymethyl]hexamethylenediamine,
[N,N-dibenzylaminoxymethyl]di-n-butylamine,
[N,N-dibenzylaminoxymethyl]di-n-hexylamine,
[N,N-dibenzylaminoxymethyl]diisopropylamine,
[N,N-diethylaminoxymethyl]di-n-butylamine,
[N,N-dibenzylaminoxymethyl]-N'-methyl-N'-2-(pyridin-2-yl)ethylamine,
[N,N-dibenzylaminoxymethyl]-N'-benzyl-N'-exo-norbornylamine or
N',N'',N'''-tris[N,N-dibenzylaminoxymethyl]hexahydro-1,3,5-triazine.

10. A composition according to claim 1, in which the organic material is a synthetic polymer.

11. A composition according to claim 10, in which the synthetic polymer is a polyolefin homopolymer or copolymer.

12. A composition according to claim 1, which additionally contains a metal salt of a higher fatty acid and/or a phenolic antioxidant.

13. A composition according to claim 12, in which the phenolic antioxidant is neopentanetetrayl tetrakis(3,5-di-tert-butyl-4-hydroxy-hydrocinnamate), n-octadecyl 3,5-di-tert-butyl-4-hydroxyhydrocinnamate, 1,3,5-trimethyl-2,4,6-tris(3,5-di-tert-butyl-4-hydroxybenzyl)benzene,
1,3,5-tris(3,5-di-tert-butyl-4-hydroxybenzyl)isocyanurate, 2,6-di-tert-butyl-p-cresol or 2,2'-ethylidene-bis(4,6-di-tert-butylphenol).

14. Use of a compound of the formula I according to claim 1 for stabilising organic material against thermal, oxidative and/or actinic degradation.

15. A compound of the formula IA,

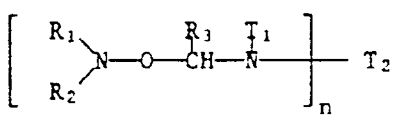

(IA)

in which n is an integer from 1 to 4, when n is 1, $R_1$ and $R_2$ are independently of one another a group of the formula (IIA)

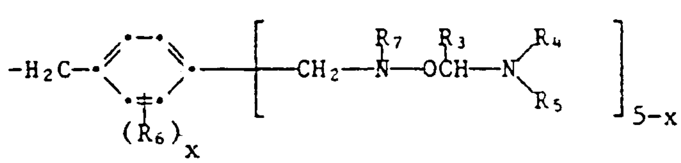

(IIA)

$R_3$, $R_4$, $R_5$, $R_6$ and $R_7$ are independently of one another hydrogen, $C_1$-$C_{36}$alkyl, $C_5$-$C_{12}$cycloalkyl, allyl, aryl, $C_7$-$C_9$aralkyl, $C_7$-$C_9$aralkyl substituted by $C_1$-$C_{36}$alkyl, bornyl or norbornyl, or $R_4$ and $R_5$ together with the nitrogen atom to which they are bonded form a 5- to 7- membered heterocyclic ring, x is an integer from 0 to 4, $T_1$ has one of the meanings given for $R_3$ or is

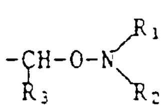

,

$T_2$ has one of the meanings given for $R_3$ or is $C_1$-$C_4$ alkyl which is substituted by a 5- to 7- membered unsaturated heterocyclic ring, or the radicals $T_1$ and $T_2$ together with the nitrogen atom to which they are bonded form a 5- to 7- membered saturated heterocyclic ring, if n is 2, 3 or 4, $R_1$ and $R_2$ are independently of one another $C_1$-$C_{36}$alkyl, $C_5$-$C_{12}$cycloalkyl, $C_7$-$C_9$aralkyl or $C_7$-$C_9$aralkyl substituted by $C_1$-$C_{36}$alkyl, or $R_1$ and $R_2$ together with the nitrogen atom to which they are bonded form a 5- to 7- membered heterocyclic ring, or $R_1$ and $R_2$ are inde-

pendently of one another a group of the formula (II)

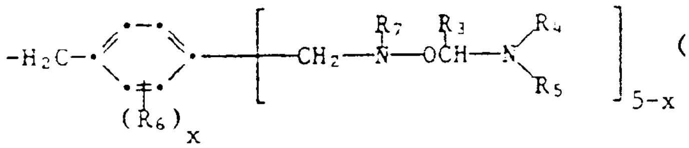 (II)

$R_3$, $R_4$, $R_5$, $R_6$ and $R_7$ are independently of one another hydrogen, $C_1$-$C_{36}$alkyl, $C_5$-$C_{12}$cycloalkyl, allyl, aryl, $C_7$-$C_9$aralkyl, $C_7$-$C_9$aralkyl substituted by $C_1$-$C_{36}$alkyl, bornyl or norbomyl, or $R_4$ and $R_5$ together with the nitrogen atom to which they are bonded form a 5- to 7 - membered heterocyclic ring, x is an integer from 0 to 5, $T_1$ has one of the meanings given for $R_3$ or is

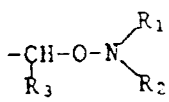 ,

when n is 2, $T_2$ is $C_2$-$C_{12}$alkylene, $C_6$-$C_{10}$cycloalkylene, $C_6$-$C_{10}$arylene or $C_6$-$C_{10}$alkylenearylenealkylene or $T_1$ and $T_2$ together with the two nitrogen atoms to which they are bonded form a 5- to 7- membered saturated heterocyclic ring,

when n is 3, $T_2$ is $C_3$-$C_6$alkanetriyl, or $T_1$ and $T_2$ together with the three nitrogen atoms to which they are bonded form a 5- to 6- membered saturated heterocyclic ring, and

when n is 4, $T_2$ is $C_4$-$C_6$alkanetetrayl.

16. The compounds (N,N-dibenzylaminoxymethyl)-N′-methyl-N′-2-(pyridin-2-yl)ethylamine and (N,N-dibenzylaminoxymethyl)-N′-benzyl-N′-exo-norbornylamine.

## Revendications

1. Composition contenant une matière organique sensible à la dégradation pouvant résulter d'une oxydation, de l'action de la chaleur et/ou de l'action d'un rayonnement actinique et au moins un composé répondant à la formule I :

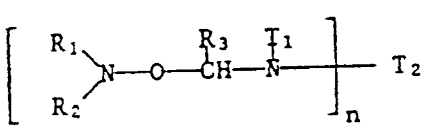 (I)

dans laquelle $R_1$ et $R_2$ représentent chacun, indépendamment l'un de l'autre, un alkyle en $C_1$-$C_{36}$, un cycloalkyle en $C_5$-$C_{12}$, un aralkyle en $C_7$-$C_9$ ou un aralkyle en $C_7$-$C_9$ porteur d'un alkyle en $C_1$-$C_{36}$, ou encore $R_1$ et $R_2$ forment ensemble, et avec l'atome d'azote auquel ils sont liés, un hétérocycle renfermant de 5 à 7 maillons, ou $R_1$ et $R_2$ représentent chacun, indépendamment l'un de l'autre, un radical répondant à la formule II :

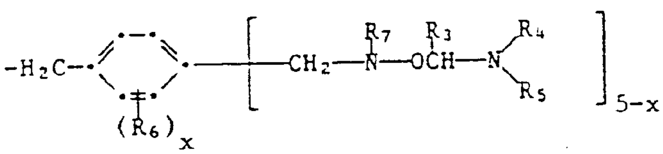 (II)

$R_3$, $R_4$, $R_5$, $R_6$ et $R_7$ représentent chacun, indépendamment les uns des autres, l'hydrogène, un alkyle en $C_1$-$C_{36}$, un cycloalkyle en $C_5$-$C_{12}$, un allyle, un aryle, un aralkyle en $C_7$-$C_9$, un aralkyle en $C_7$-$C_9$ porteur d'un alkyle en $C_1$-$C_{36}$, un bornyle ou un norbornyle, ou $R_4$ et $R_5$ forment ensemble, et avec l'atome d'azote auquel ils sont liés, un hétérocycle renfermant de 5 à 7 maillons, x désigne un nombre entier de 0 à 5, n désigne un nombre entier de 1 à 4, $T_1$ a l'une des significations indiquées pour $R_3$ ou représente un radical

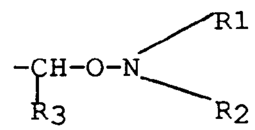

,

et

T2 représente lorsque n est égal à 1, l'un des radicaux indiqués pour $R_3$ ou un alkyle en $C_1$-$C_4$ porteur d'un hétérocycle insaturé renfermant de 5 à 7 maillons, ou forme, avec $T_1$ et l'atome d'azote auquel $T_1$ et $T_2$ sont liés, un hétérocycle saturé renfermant de 5 à 7 maillons,

lorsque n est égal à 2, un alkylène en $C_2$-$C_{12}$, un cycloalkylène en $C_6$-$C_{10}$, un arylène en $C_6$-$C_{10}$ ou un alkylène-arylène-alkylène en $C_8$-$C_{10}$, ou forme, avec $T_1$ et avec les deux atomes d'azote auxquels $T_1$ et $T_2$ sont liés, un hétérocycle saturé renfermant de 5 à 7 maillons,

lorsque n est égal à 3, un alcane-triyle en $C_3$-$C_6$, ou forme, avec $T_1$ et avec les trois atomes d'azote auxquels $T_1$ et $T_2$ sont liés, un hétérocycle saturé renfermant 5 ou 6 maillons, et

lorsque n est égal à 4, un alcane-tétrayle en $C_4$-$C_6$.

2. Composition selon la revendication 1 dans laquelle $R_1$ à $R_7$ représentent chacun, indépendamment les uns des autres, un alkyle en $C_1$-$C_{18}$, un cyclopentyle, un cyclohexyle, un benzyle, un $\alpha$-méthylbenzyle ou un $\alpha$,$\alpha$-diméthylbenzyle et $R_3$ peut en outre représenter l'hydrogène.

3. Composition selon la revendication 1 dans laquelle $R_1$ et $R_2$ représentent chacun un benzyle et $R_3$ représente l'hydrogène.

4. Composition selon la revendication 1 dans laquelle n est égal à 1, $T_1$ et $T_2$ représentent chacun, indépendamment l'un de l'autre, un alkyle en $C_1$-$C_{18}$, un cyclopentyle, un cyclohexyle, un allyle, un phényle, un benzyle, un $\alpha$-méthylbenzyle ou un $\alpha$,$\alpha$-diméthylbenzyle, ou $T_1$ et $T_2$ forment ensemble, et avec l'atome d'azote auxquels ils sont liés,un radical morpholino, pyrrolidino ou pipéridino.

5. Composition selon la revendication 1 dans laquelle n est égal à 2, $T_2$ représente un alkylène en $C_2$-$C_6$, un phénylène ou un xylylène, ou $T_1$ et $T_2$ forment ensemble, et avec les deux atomes d'azote auxquels ils sont liés, un radical de pipérazine.

6. Composition selon la revendication 1 dans laquelle n est égal à 3, $T_2$ représente un radical glycéryle ou triméthylyl-propane, ou $T_1$ et $T_2$ forment ensemble, et avec les trois atomes d'azote auxquels ils sont liés, un cycle d'hexahydro-triazine-1,3,5.

7. Composition selon la revendication 1 dans laquelle n est égal à 4, et $T_2$ représente un radical pentaérythrityle.

8. Composition selon la revendication 1 dans laquelle $R_1$ et $R_2$ représentent chacun, indépendamment l'un de l'autre, un alkyle en $C_1$-$C_6$ ou un benzyle, $R_3$ représente l'hydrogène, n est égal à 1, à 2 ou à 3 et, lorsque n est égal à 1, $T_1$ et $T_2$ représentent chacun, indépendamment l'un de l'autre, un alkyle en $C_1$-$C_8$, un allyle, un phényle, un benzyle ou un norbornyle et $T_2$ peut en outre représenter un alkyle en $C_1$-$C_4$ porteur d'un radical pyridyle, ou $T_1$ et $T_2$ forment ensemble, et avec l'atome d'azote auquel ils sont liés, un radical pipéridino, lorsque n est égal à 2 $T_1$ représente un alkyle en $C_1$-$C_6$ et $T_2$ un alkylène en $C_2$-$C_6$ ou $T_1$ et $T_2$ forment ensemble, et avec les deux atomes d'azote auxquels ils sont liés, un cycle de pipérazine, et, lorsque n est égal à 3, $T_1$ et $T_2$ forment ensemble, et avec les trois atomes d'azote auxquels ils sont liés, un radical d'hexahydro-triazine-1,3,5.

9. Composition selon la revendication 1 dans laquelle le composé de formule I est :
1 la [N,N-dibenzylaminoxy-méthyl]-1,4 pipérazine,
2 la N′,N″-diméthyl-N′,N″-bis-[N,N-dibenzylaminoxyméthyl]-hexaméthylène-diamine,
3 la [N,N-dibenzylaminoxy-méthyl]-di-n-butylamine,
4 la [N,N-dibenzylaminoxy-méthyl]-di-n-hexylamine,
5 la [N,N-dibenzylaminoxy-méthyl]-di-isopropylamine,
6 la [N,N-diéthylaminoxy-méthyl]-di-n-butylamine,
7 la [N,N-dibenzylaminoxy-méthyl]-N′-méthyl-N′-[(pyridyl-2)-2 éthyl]-amine,
8 la [N,N-dibenzylaminoxy-méthyl]-N′-benzyl-N′-exo-norbornyl-amine ou
9 la N′,N″,N‴-tris-[N,N-dibenzylaminoxy-méthyl]-hexahydro-triazine-1,3,5.

10. Composition selon la revendication 1 dans laquelle la matière organique est un polymère synthétique.

11. Composition selon la revendication 10 dans laquelle le polymère synthétique est un homopolymère ou un copolymère d'oléfines.

12. Composition selon la revendication 1 qui contient en outre un sel métallique d'un acide gras supérieur et/ou un anti-oxydant phénolique.

13. Composition selon la revendication 12 caractérisée en ce que l'anti-oxydant phénolique est le tétrakis-(di-tert-butyl-3,5 hydroxy-4 hydrocinnamate) de néopentane-tétrayle, le di-tert-butyl-3,5 hydroxy-4 hydrocinnamate de n-octadécyle, le triméthyl-1,3,5 tris-(di-tert-butyl-3,5 hydroxy-4 benzyl)-2,4,6 benzène, l'isocyanurate de tris-(di-tert-butyl-3,5 hydroxy-4 benzyle), le di-tert-butyl-2,6 p-crésol, ou l'éthylidène-2,2 bis-(di-tert-butyl-4,6 phénol).

14. Application de composés de formule I selon la revendication 1 à la stabilisation d'une matière organique contre la dégradation thermique, oxydative et/ou actinique.

15. Composés répondant à la formule IA :

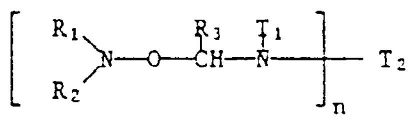

(IA)

dans laquelle n désigne un nombre entier de 1 à 4 et lorsque n est égal à 1, $R_1$ et $R_2$ représentent chacun, indépendamment l'un de l'autre, un radical de formule IIA :

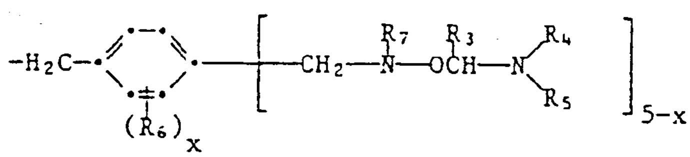

(IIA)

$R_3$, $R_4$, $R_5$, $R_6$ et $R_7$ représentent chacun, indépendamment les uns des autres, l'hydrogène, un alkyle en $C_1$-$C_{36}$, un cycloalkyle en $C_5$-$C_{12}$, un allyle, un aryle, un aralkyle en $C_7$-$C_9$, un aralkyle en $C_7$-$C_9$ porteur d'un alkyle en $C_1$-$C_{36}$, un bornyle ou un norbornyle, ou $R_4$ et $R_5$ forment ensemble, et avec l'atome d'azote auquel ils sont liés, un hétérocycle renfermant de 5 à 7 maillons, x représente un nombre entier de 0 à 5, $T_1$ a l'une des significations indiquées pour $R_3$ ou représente un radical

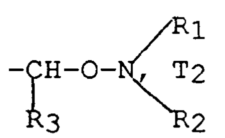

a l'une des significations qui ont été données pour $R_3$ ou représente un alkyle en $C_1$-$C_4$ porteur d'un hétérocycle insaturé renfermant de 5 à 7 maillons, ou $T_1$ et $T_2$ forment ensemble, et avec l'atome d'azote auquel ils sont liés, un hétérocycle saturé renfermant de 5 à 7 maillons, lorsque n est égal à 2, à 3 ou à 4, $R_1$ et $R_2$ représentent chacun, indépendamment l'un de l'autre, un alkyle en $C_1$-$C_{36}$, un cycloalkyle en $C_5$-$C_{12}$, un aralkyle en $C_7$-$C_9$ ou un aralkyle en $C_7$-$C_9$ porteur d'un alkyle en $C_1$-$C_{36}$, ou encore $R_1$ et $R_2$ forment ensemble, et avec l'atome d'azote auquel ils sont liés, un hétérocycle renfermant de 5 à 7 maillons, ou $R_1$ et $R_2$ représentent chacun, indépendamment l'un de l'autre, un radical répondant à la formule II :

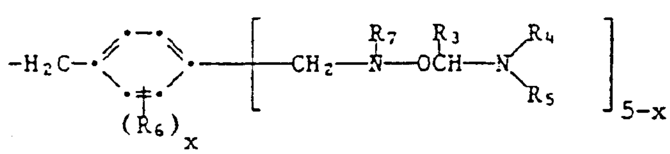

(II)

$R_3$, $R_4$, $R_5$, $R_6$ et $R_7$ représentent chacun, indépendamment les uns des autres, l'hydrogène, un alkyle en $C_1$-$C_{36}$, un cycloalkyle en $C_5$-$C_{12}$, un allyle, un aryle, un aralkyle en $C_7$-$C_9$, un aralkyle en $C_7$-$C_9$ porteur d'un alkyle en $C_1$-$C_{36}$, un bornyle ou un norbornyle, ou $R_4$ et $R_5$ forment ensemble, et avec l'atome d'azote auquel ils sont liés, un hétérocycle renfermant de 5 à 7 maillons, x représente un nombre entier de 0 à 5, $T_1$ a l'une des significations qui ont été indiquées pour $R_3$ ou représente un radical

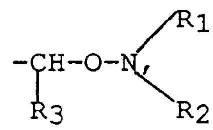

lorsque n est égal à 2, $T_2$ représente un alkylène en $C_2$-$C_{12}$, un cycloalkylène en $C_6$-$C_{10}$, un arylène en $C_6$-$C_{10}$ ou un alkylène-arylène-alkylène en $C_8$-$C_{10}$, ou $T_1$ et $T_2$ forment ensemble, et avec les deux atomes d'azote auxquels ils sont liés, un hétérocycle saturé renfermant de 5 à 7 maillons,

lorsque n est égal à 3, $T_2$ représente un alcane-triyle en $C_3$-$C_6$, ou $T_1$ et $T_2$ forment ensemble, et avec les trois atomes d'azote auxquels ils sont liés, un hétérocycle saturé renfermant 5 ou 6 maillons, et

lorsque n est égal à 4, $T_2$ représente un alcane-tétrayle en $C_4$-$C_6$.

16. Les composés suivants : la (N,N-dibenzylaminoxy-méthyl)-N′-méthyl-N′-[(pyridyl-2)-2-éthyl]-amine et la (N,N-dibenzylaminoxy-méthyl)-N′-benzyl-N′-exo-norbornylamine.